# EUROPEAN PATENT SPECIFICATION

(11) **EP 3 068 220 B1**
(45) Date of publication and mention of the grant of the patent: **24.10.2018**
(21) Application number: 14793167.9
(22) Date of filing: 05.11.2014
(51) Int. Cl.: A01N 25/30, C08F 271/02, A01P 3/00, C08F 290/06

(54) **A COMPOSITION COMPRISING AN ACTIVE AND A GRAFT COPOLYMER MADE OF N-VINYLLACTAM, VINYL ESTER, AND AN ALCOHOL ALKOXYLATE**
ZUSAMMENSETZUNG ENTHALTEND EINEN WIRKSTOFF UND EIN PFROPFCOPOLYMER AUS N-VINYLLACTAM, VINYLESTER, UND ALKOHOLALKOXYLAT
COMPOSITION COMPRENANT DE PRINCIPE ACTIF ET DE COPOLYMÈRE GREFFÉ CONSTITUÉ DE N-VINYLLACTAME, UN ESTER VINYLIQUE ET UN ALCOXYLATE D'ALCOOL

(30) Priority: 15.11.2013 EP 13193134
(43) Date of publication of application: 21.09.2016
(73) Proprietor: BASF SE, 67056 Ludwigshafen am Rhein (DE)
(72) Inventor: DIECKMANN, Yvonne, 67454 Haßloch (DE); GUTZLER, Rainer, 67373 Dudenhofen (DE); MERTOGLU, Murat, 67063 Ludwigshafen (DE); MARGUERRE, Ann-Kathrin, 69121 Heidelberg (DE); BERGHAUS, Rainer, 67346 Speyer (DE)
(74) Representative: BASF IP Association
(86) International application number: PCT/EP2014/073758
(87) International publication number: WO 2015/071139

(56) References cited:
- WO-A1-2011/121477
- US-A1- 2012 178 728

## Description

The present invention relates to a composition comprising an active and a graft copolymer obtainable by free-radical polymerization of a monomer mixture comprising N-vinyllactam, vinyl ester, and an alcohol alkoxylate, wherein the N-vinyl lactam is N-vinyl pyrrolidon. It further relates to a method for preparing said composition by mixing the pesticide and the graft copolymer and to a method for con-trolling phytopathogenic fungi and/or undesired plant growth and/or undesired attack by insects or mites and/or for regulating the growth of plants. Combinations of preferred embodiments with other preferred embodiments are within the scope of the present invention.
Besides the optimization of the active ingredient properties, the development of an effective agent is of particular importance with regard to the industrial production and application of active ingredients. By formulating the active ingredients correctly, an optimal balance must be found between properties, some of which are in conflict with each other, such as the biological activity, the toxicology, potential environmental effects, and the costs. Moreover, the formulation is a decisive factor in determining the shelf life and the user friendliness of a composition.
An efficient uptake of the active ingredient by the plant is of particular importance for the activity of an agrochemical composition. If this uptake is via the leaf, it constitutes a complex translocation process, where the active substance, for example a herbicide, must first penetrate the waxy cuticula of the leaf and subsequently diffuse, via the cuticula, into the tissue underneath, to the actual site of action.
Copolymers obtainable by free-radical polymerization of N-vinyllactam, vinyl acetate and a polyether are known:
WO2007/051743 discloses the use of a copolymers obtained by free-radical polymeri-zation of N-vinyllactam, vinyl acetate and a polyether as solubilizer for in water insoluble compounds, such as agrochemical active ingredients.
WO2011/121477 discloses the use of a copolymers obtained by free-radical polymerization of N-vinyllactam, vinyl acetate and a polyether for increasing the activity of a pesticide.
Problems associated with known adjuvants are that they often decrease the stability of the formulation, especially of suspension formulations, e.g. by increasing the viscosity or the particle size. Many known adjuvants have a high phytotoxicity, so their use is not advisable for treating vines, fruits and vegetables. There are also adjuvants, which show adverse effects on the skin of the farmers, or which are too expensive to synthesize on industrial scale. Object of the present invention was to overcome these aforementioned problems.

The object was solved by a composition comprising an active and a graft copolymer obtainable by free-radical polymerization of a monomer mixture comprising N-vinyllactam, vinyl ester, and an alcohol alkoxylate, wherein the N-vinyl lactam is N-vinyl pyrrolidon. The object was also solved by a graft copolymer obtainable by free-radical polymerization of a monomer mixture comprising N-vinyllactam, vinyl ester, and an alcohol alkoxylate, wherein the N-vinyl lactam is N-vinyl pyrrolidon.

A **graft polymer** is a well known type of polymer structure, which typically comprises a main polymer chain connected to polymeric side chains. The graft copolymer is obtainable (preferably it is obtained) by free-radical polymerization of a monomer mixture comprising N-vinyllactam, vinyl ester, and an alcohol alkoxylate, wherein the N-vinyl lactam is N-vinyl pyrrolidon. Such free-radical polymerizations of monomer mixtures yielding graft copolymers are well known as laid out below.

Examples of suitable **vinyl esters** include vinyl esters of saturated carboxylic acids having 1 to 20, especially 1 to 6, carbon atoms. Examples are vinyl acetate, vinyl propionate, vinyl butanoate, vinyl hexanoate and/or vinyl octanoate. It is preferred to use vinyl acetate, vinyl propionate, or mixutres thereof. Particular preference is given to using vinyl acetate. In accordance with the invention it is possible to use one vinyl ester alone or a mixture of two or more vinyl esters.

Suitable **N-vinyl lactam** is N- vinyl pyrrolidone. In accordance with the invention it is possible to use one N-vinyl lactam alone or a mixture of two or more N-vinyl lactams.

Suitable **alcohol alkoxylates** may be obtainable by alkoxylation of alcohols with alkylene oxide, such as ethylene oxide.

The alcohol alkoxylate may comprise 1 to 200, preferably 3 to 100, and in particular 5 to 90 units of alkoxylate. In general, the units of alkoxylate correspond to the molar ratio of alkylene oxide used for alkoxylation of the alcohol.

The alkoxylate unit of the alcohol alkoxylate may comprise ethoxylate, and optionally in addition propoxylate, butoxylate, pentoxylate, or mixtures thereof. Preferably, the alkoxylate unit of the alcohol alkoxylate comprises ethoxylate, and optionally in addition propoxylate. In particular, the alcohol alkoxylate is an alcohol ethoxylate. The alkoxylate unit of the alcohol alkoxylate may be a random or block copolymers. The block copolymers may be of the AB or ABA type.

The alcohol alkoxylate is usually based on a linear or branched, saturated or unsaturated C₈-C₃₄ alcohol, preferably a C₁₀-C₂₂ alcohol, and in particular a C₁₂-C₁₈ alcohol.
The alcohol alkoxylates may have molecular weights Mn of from 200 to 100 000 D [daltons], preferably 300 to 20 000 D, particularly preferably 400 to 10 000 D. In another preferred form the alcohol alkoxylates has a molecular weight Mn of from 300 to 1500 D, or from 350 to 900 D. The molecular weights are determined on the basis of the OH number measured as specified in DIN 53240.

In a preferrred form the alcohol alkoxylate comprises 3 to 100 units of alkoxylate, the alkoxylate unit of the alcohol alkoxylate comprises ethoxylate (optionally in addition propoxylate), and the alcohol alkoxylate is based on a linear or branched, saturated or unsaturated C₈-C₃₄ alcohol.

In a more preferrred form the alcohol alkoxylate comprise of 5 to 90 units of alkoxylate, the alkoxylate unit of the alcohol alkoxylate is ethoxylate, and the alcohol alkoxylate is based on a linear or branched, saturated or unsaturated C₁₀-C₂₂ alcohol.

The monomer mixture may comprise an **additional comonomer**.

Examples of suitable additional comonomers are vinylcarboxamides such as N-vinylformamide, N-vinyl-N-methylformamide, N-vinylacetamide, N-vinyl-N-methylacetamide, N-vinyl-N-methylpropionamide, and N-vinylpropionamide. It is pre-ferred to use N-vinylformamide and/or N-vinyl-N-methylacetamide. The copolymerized monomer units of N-vinylformamide and/or N-vinyl-N-methylacetamide may be partly or fully hydrolyzed.

Suitable further additional comonomers are also monoethylenically unsaturated monocarboxylic and dicarboxylic acids or their anhydrides having 3 to 6 carbon atoms, such as acrylic acid, methacrylic acid, crotonic acid, maleic acid or anhydride, fumaric acid, itaconic acid or anhydride, and citraconic acid or anhydride.

Further suitable additional comonomers are the amides, esters, and nitriles of the aforementioned monoethylenically unsaturated C₃ to C₆ carboxylic acids, such as, for example, the amides acrylamide, methacrylamide, and also N-alkyl- and N,N-dialkylamides having alkyl radicals of 1 to 6 carbon atoms, such as N-methylacrylamide, N,N-dimethylacrylamide, N-methylmethacrylamide, N,N-dimethylmethacrylamide, N-ethylacrylamide, N-propylacrylamide, tert-butylacrylamide and tert-butylmethacrylamide, and also the basic (meth)acrylamides, such as 2-N,N-dimethylaminoethylacrylamide, 2-N,N-dimethylaminoethylmethacrylamide, 2-N,N-diethylaminoethylacrylamide, 2-N,N-diethylaminoethylmethacrylamide, 3-N,N-dimethylaminopropylacrylamide, 3-N,N-diethylaminopropylacrylamide, 3-N,N-dimethylaminopropylmethacrylamide and 3-N,N-diethylaminopropylmethacrylamide.

Other suitable additional comonomers are the esters of monoethylenically unsaturated carboxylic acids with C1 to C6 alcohols, such as methyl acrylate, methyl methacrylate, ethyl acrylate, and ethyl methacrylate, or with glycols or polyglycols, in each case only one OH group in the glycols and polyglycols being esterified with an ethylenically un-saturated carboxylic acid, such as hydroxyethyl acrylate, hydroxyethyl methacrylate, hydroxypropyl acrylates, hydroxybutyl acrylates, hydroxypropyl methacrylates, hy-droxybutyl methacrylates, and also the (meth)acrylic monoesters of polyalkylene glycols with a molar weight of 200 to 10 000. Of further suitability are the esters of the aforemen-tioned ethylenically unsaturated carboxylic acids with pyrrolidone derivatives, such as, for example, 2-(N-pyrrolidone)ethyl acrylate or 2-(N-pyrrolidone)ethyl methacrylate, and with amino alcohols, such as 2-N,N-dimethylaminoethyl acrylate, 2-N,N-dimethylaminoethyl methacrylate, 2-N,N-diethylaminoethyl acrylate, 2-N,N-diethylaminoethyl methacrylate, 3-N,N-dimethylaminopropyl acrylate, 3-N,N-dimethylaminopropyl methacrylate, 3-N,N-diethylaminopropyl acrylate, 3-N,N-diethylaminopropyl methacrylate, 4-N,N-dimethylaminobutyl acrylate, 4-N,N-diethylaminobutyl acrylate, 5-N,N-dimethylaminopentyl acrylate, dimethylaminoneopentyl methacrylate and 6-N,N-dimethylaminohexyl acrylate. The basic (meth)acrylates and (meth)acrylamides are used in the form of the free bases, of the salts with mineral acids, such as hydrochloric acid, sulfuric acid, and nitric acid, or in quaternized form. Examples of suitable quaternizing agents include dimethyl sulfate, methyl chloride, ethyl chloride, benzyl chloride or diethyl sulfate.

Examples of nitriles of the aforementioned ethylenically unsaturated carboxylic acid are acrylonitrile and methacrylonitrile.

Additionally suitable as additional comonomers are N-vinyl imidazole and also substi-tuted N-vinyl imidazoles, such as N-vinyl-2-methylimidazole, N-vinyl-4-methylimidazole, N-vinyl-5-methylimidazole, and N-vinyl-2-ethylimidazole, N-vinyl imidazolines, such as N-vinyl imidazoline, N-vinyl-2-methylimidazoline, and N-vinyl-2-ethylimidazoline, and also N-vinyl imidazolidinones, such as N-vinyl-2-imidazolidinone and N-vinyl-4-methyl-2-imidazolidinone. N-Vinyl imidazoles, N-vinyl imidazolines, and N-vinyl imidazolidinones are used not only in the form of the free bases but also in a form neutralized with mineral acids or in quaternized form, the quaternization being performed preferably using dimethyl sulfate, diethyl sulfate, benzyl chloride, methyl chloride or ethyl chloride.

Finally, monomers suitable as additional comonomers include those comprising sulfo groups, such as vinylsulfonic acid, allylsulfonic acid, methallylsulfonic acid, styrenesul-fonic acid, 3-sulfopropyl acrylate, 3-sulfopropyl methacrylate, and 2-acrylamido-2-methylpropanesulfonic acid. The compounds containing acid groups can be used in the form of the free acids, the ammonium salts or the alkali metal and alkaline earth metal salts for the graft polymerization.

Additional comonomers may also be monomers having a crosslinking action, such as, for example, methylenebisacrylamide, esters of acrylic acid and methacrylic acid with polyhydric alcohols, examples being glycol diacrylate, glycerol triacrylate, glycol di-methacrylate, and glycerol trimethacrylate, and also polyols, such as pentaerythritol and glucose, which are esterified at least doubly with acrylic acid or methacrylic acid. Further suitable crosslinkers are divinylbenzene, divinyldioxane, N,N-divinyl-2-imidazolidinone, pentaerythritol triallyl ether, and pentaallylsucrose. Preferred crosslinking monomers c) are water-soluble monomers, such as glycol diacrylate or glycol diacrylates of polyethylene glycols with a molecular weight (numerical average) of 300 to 10 000.
Among the additional comonomers N-vinyl imidazole, acrylic acid, methacrylic acid, methacrylamide, N,N-dimethylacrylamide, N-methylmethacrylamide, tert-butylacrylamide, tert-butylmethacrylamide, dimethylaminoethylmethacrylamide, hydroxyethyl acrylate, 2-(N-pyrrolidone)ethyl acrylate, 2-(N-pyrrolidone)ethyl methacrylate, and 2-acrylamido-2-methylpropanesulfonic acid are preferably employed. Advantageously in accordance with the invention, however, no monomers c) are employed.

According to one embodiment of the invention, suitable graft copolymers are obtainable from a monomer mixture comprising
i) 5 to 60% by weight of N-vinyllactam (N-vinyl pyrrolidone),
ii) 5 to 60% by weight of vinyl ester (e.g. vinyl acetate),
iii) 10 to 80% by weight of alcohol alkoxylate (e.g. C₈₋₂₂ alcohol ethoxylate), and
iv) up to 20%, preferably up to 10 % by weight of additional comonomers,
wherein the sum of component i) to iv) adds up to 100 %.

Preferred graft copolymers are obtainable from:
i) 10 to 45% by weight of N-vinyllactam (N-vinyl pyrrolidone),
ii) 10 to 45% by weight of vinyl ester (e.g. vinyl acetate),
iii) 15 to 80% by weight of a alcohol alkoxylate (e.g. C₈₋₂₂ alcohol ethoxylate), and
iv) up to 10% by weight of additional comonomers,
wherein the sum of component i) to iv) adds up to 100 %.

In another preferred form graft copolymers are obtainable from:
i) 20 to 60%, preferably 30 to 50% by weight of N-vinyllactam (N-vinyl pyrrolidone),
ii) 20 to 60%, preferably 30 to 50% by weight of vinyl ester (e.g. vinyl acetate),
iii) 10 to 50%, preferably 15 to 35% by weight of a alcohol alkoxylate (e.g. C₈₋₂₂ alcohol ethoxylate), and
iv) up to 20%, preferably up to 10% by weight of additional comonomers,
wherein the sum of component i) to iv) adds up to 100 %.

In another preferred form graft copolymers are obtainable from:
i) 5 to 40%, preferably 10 to 30% by weight of N-vinyllactam (N-vinyl pyrrolidone),
ii) 5 to 40%, preferably 10 to 30% by weight of vinyl ester (e.g. vinyl acetate),
iii) 35 to 80%, preferably 45 to 75% by weight of a alcohol alkoxylate (e.g. C₈₋₂₂ alcohol ethoxylate), and
iv) up to 20%, preferably up to 10% by weight of additional comonomers,
wherein the sum of component i) to iv) adds up to 100 %.

Usually, the components i), ii), iii) and optionally the amount of the additional comono-mers add up to 100 % in the monomer mixture. Preferably, the components i), ii), and iii) add up to 100 % in the monomer mixture. It will be appreciated that mixtures of two or more additional comonomers can also be used.

The amount of the graft polymer is usually in the range of from 5 to 1000 wt%, preferably from 10 to 500 wt%, more preferably from 20 to 100 wt%, based on the weight of the active.

The composition may comprise 1 to 60 wt%, preferably 5 to 40 wt%, more preferably 8 to 30 wt%, and in particular 10 to 20 wt% of the graft polymer.

General processes for **preparing the graft copolymers** are known per se (e.g. from WO 2007/051743). The preparation takes place by free-radical polymerization, preferably solution polymerization, in nonaqueous organic solvents or in mixed nonaque-ous/aqueous solvents. The polymerization is preferably carried out at temperatures from 60 to 100°C. Suitable nonaqueous organic solvents are, for example, alcohols such as methanol, ethanol, n-propanol and isopropanol, and glycols such as ethylene glycol and glycerol. Further suitable solvents are esters such as, for example, ethyl acetate, n-propyl acetate, isopropyl acetate, isobutyl acetate or butyl acetate.

Free-radical **initiators** are employed to initiate the polymerization. The amounts of initiator or initiator mixtures used, based on monomer employed, are between 0.01 and 10% by weight, preferably between 0.3 and 5% by weight. Depending on the nature of the solvent used, both organic and inorganic peroxides are suitable, such as sodium per-sulfate or azo initiators such as azobisisobutyronitrile, azo-bis(2-amidopropane) dihy-drochloride or 2,2'-azobis(2-methylbutyronitrile). Examples of peroxide initiators are dibenzoyl peroxide, diacetyl peroxide, succinyl peroxide, tert-butyl perpivalate, tert-butyl perethylhexanoate, tert-butyl perneodecanoate, tert-butyl permaleate, bis-(tert-butylper)cyclohexane, tert-butylperisopropyl carbonate, tert-butyl peracetate, 2,2-bis(tert-butylper)butane, dicumyl peroxide, di-tert-amyl peroxide, di-tert-butyl peroxide, p-menthane hydroperoxide, pinane hydroperoxide, cumene hydroperoxide, tert-butyl hydroperoxide, hydrogen peroxide and mixtures of said initiators. Said initiators can also be used in combination with redox components such as ascorbic acid. Particularly suitable initiators are tert-butyl perneodecanoate, tert-butyl perpivalate or tert-butyl per-ethylhexanoate.

The free-radical polymerization can place if appropriate in the presence of emulsifiers, if appropriate further protective colloids, if appropriate molecular weight regulators, if appropriate buffer systems and if appropriate subsequent pH adjustment using bases or acids.

Suitable **molecular weight regulators** are sulfhydryl compounds such as alkyl mercap-tans, e.g. n-dodecyl mercaptan, tert-dodecyl mercaptan, thioglycerol, thioglycolic acid and esters thereof (e.g. 2-ethylhexyl thioglycolate), mercaptoalkanols such as mercaptoethanol. Further suitable regulators are mentioned for example in DE 197 12 247 A1, page 4. The necessary amount of the molecular weight regulators is in the range from 0 to 5% by weight based on the amount of (co)monomers to be polymerized. If regulators are used, the amount employed is in particular in the range from 0.05 to 2% by weight, particularly preferably 0.1 to 1.5% by weight. However, polymerization in the absence of a regulator is very particularly preferred.

It is also possible if appropriate to use emulsifiers, for example ionic or nonionic surfac-tants whose HLB is normally in the range from 3 to 13. For the definition of HLB, refer-ence is made to the publication by W.C. Griffin, J. Soc. Cosmetic Chem., Volume 5, 249 (1954). The amount of surfactants based on the polymer can be from 0 to 10% by weight, preferably 0 to 5% by weight.

The monomer, or the monomer mixture or the emulsion of monomer(s) are introduced together with the initiator, which is generally present in solution, into a stirred reactor at the polymerization temperature (batch process) or if appropriate metered continuously or in a plurality of consecutive stages into the polymerization reactor (feed process). It is usual in the feed process for the reactor to be charged, before the start of the actual polymerization, besides the solvent (in order to make stirring of the reaction mixture possible) also with partial quantities, rarely the total quantity intended for the polymeri-zation, of the starting materials such as emulsifiers, protective colloids, monomers, regulators etc. or partial quantities of the feeds (generally monomer feed or emulsion feed and initiator feed).

The polymerization can be carried out both under atmospheric pressure and in a closed reactor under elevated pressure. In this case it is possible to polymerize either under the pressure set up during the reaction, or the pressure can be adjusted by injecting a gas or evacuating. The pressure can also be controlled by partial decompression of the reactor into the condenser.

A nonaqueous solvent used for the polymerization can subsequently be removed and replaced by water by steam distillation. This normally entails initially the nonaqueous solvent being distilled out pure as far as possible and then being completely replaced by water by passing in steam.

After the polymerization it is possible to employ generally known processes for reducing residual monomers. Examples of such processes are further addition of an initiator at the end of the polymerization, hydrolysis of vinyllactam monomers by adding acids, treatment of the polymer solution with solid phases such as ion exchangers, feeding in a monomer which copolymerizes well, membrane filtration and further customary methods.

The solids content of the resulting aqueous polymer dispersions or solutions is usually from 10 to 70% by weight, preferably 15 to 60% by weight, particularly preferably 15 to 40% by weight.

The polymer dispersions or solutions can be converted into powder form or into granules by various drying processes such as, for example, spray drying, fluidized spray drying, drum drying, paddle drying, belt drying or freeze drying. It may be advisable during the spray drying to add additives such as, for example, colloidal silica or hydrophobically modified colloidal silica. The copolymers are obtained as aqueous dispersions or aqueous solutions or, after removal of the water content, as very free-flowing, water-dispersible or water-soluble powders.

Examples of **actives** (also termed active ingredients) are active agrochemical ingredients, active cosmetic ingredients, active pharmaceutical ingredients or food supplements (such as vitamins or carotenoids). Preferred active ingredients are active agrochemical ingredients (also termed pesticides).

Examples of active **cosmetic** ingredients are cosmetic oils, flavorings and aromas, vitamins or UV absorbers. Cosmetic oils include groundnut oil, jojoba oil, coconut oil, almond oil, olive oil, palm oil, castor oil, soybean oil, wheatgerm oil, or essential oils such as mountain pine oil, lavender oil, rosemary oil, spruce needle oil, pine needle oil, eucalyptus oil, peppermint oil, sage oil, bergamot oil, terpentine oil, melissa oil, juniper oil, lemon oil, anise oil, cardamom oil, camphor oil, etc., or mixtures thereof. UV absorbers include 2-hydroxy-4-methoxybenzophenone, 2,2',4,4'-tetrahydroxybenzo-phenone, 2,2'-dihydroxy-4,4'-dimethoxybenzophenone, 2,4-dihydroxybenzophenone, 2'-ethylhexyl 2-cyano-3,3-diphenylacrylate, 2,4,6-trianilino-p-(carbo-2'-ethylhexyl-1'-oxy)-1,3,5-triazine, 3-(4-methoxybenzylidene)camphor, 2-ethylhexyl N,N-dimethyl-4-amino-benzoate, 3,3,5-trimethylcyclohexyl salicylate, 4-isopropyldibenzoylmethane, 2 ethylhexyl p-methoxycinnamate and 2-isoamyl p-methoxycinnamate, and mixtures thereof.

Examples of **flavorings** and aromas are as described in WO 01/49817, or in "Flavors and Fragrances", Ullmann's Encyclopedia of Industrial Chemistry, Wiley-VCH, 2002, to which explicit reference is made.

Examples of **vitamins** are vitamins, provitamins and vitamin precursors from groups A, C, E and F, especially 3,4-didehydroretinol, beta-carotene (provitamin of vitamin A), ascorbic acid (vitamin C), and the palmitic esters, glucosides or phosphates of ascorbic acid, tocopherols, especially alpha-tocopherol and esters thereof, for example the acetate, the nicotinate, the phosphate and the succinate; and additionally vitamin F, which is understood to mean essential fatty acids, particularly linolic acid, linolenic acid and arachidonic acid.

Examples of **pharmaceutical actives** include: benzodiazepines, antihypertensives, vitamins, cytostatics, especially taxol, anesthetics, neuroleptics, antidepressives, antiviral agents, for example anti-HIV agents, antibiotics, antimycotics, antidementia drugs, fungicides, chemotherapeutics, urologics, platelet aggregation inhibitors, sulfonamides, spasmolytics, hormones, immunoglobulins, sera, thyroid therapeutics, psychoactive drugs, Parkinson's drugs and other antihyperkinetics, ophthalmics, neuropathy preparations, calcium metabolism regulators, muscle relaxants, lipid-lowering drugs, hepatotherapeutics, coronary drugs, cardiac drugs, immunotherapeutics, regulatory peptides and inhibitors thereof, hypnotics, sedatives, gynecologicals, gout remedies, fibrinolytics, enzyme preparations and transport proteins, enzyme inhibitors, emetics, blood flow stimulators, diuretics, diagnostic agents, corticoids, cholinergics, biliary therapeutics, antiasthmatics, bronchodilators, beta receptor blockers, calcium antagonists, ACE inhibitors, arteriosclerosis drugs, antiinflammatories, anticoagulants, antihypotensives, antihypoglycaemics, antihypertensives, antifibrinolytics, antiepileptics, antiemetics, antidotes, antidiabetics, antiarrhythmics, antianaemics, antiallergics, anthelmintics, analgesics, analeptics, aldosterone antagonists, slimming agents.
Actives, such as pharmaceutical actives are preferably hydrophobic actives. The term hydrophobic actives means that the active has a solubility in water at 20 °C of less than 0.25 % (m/m), preferably less than 0.1 %, in particular less than 0.01 %.

The term **pesticides** refers to at least one active substance selected from the group of the fungicides, insecticides, nematicides, herbicides, safeners, biopesticides and/or growth regulators. Preferred pesticides are fungicides, insecticides, herbicides and growth regulators. Especially preferred pesticides are fungicides. Mixtures of pesticides of two or more of the abovementioned classes may also be used. The skilled worker is familiar with such pesticides, which can be found, for example, in the Pesticide Manual, 16th Ed. (2013), The British Crop Protection Council, London. The following classes A) to K) refer to fungicides.
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxystrobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)-phenyl)-2-methoxyimino-N-methylacetamide, pyribencarb, triclopyricarb/chlorodincarb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate
   - inhibitors of complex II (e. g. carboxamides): benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, N-(7-fluoro-1,1,3-trimethyl-indan-4-yl)-1,3-dimethyl-pyrazole-4-carboxamide, N-[2-(2,4-dichlorophenyl)-2-methoxy-1-methyl-ethyl]-3-(difluoromethyl)-1-methyl-pyrazole-4-carboxamide;
   - other respiration inhibitors (e.g. complex I, uncouplers): diflumetorim, (5,8-difluoroquinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazolo, 2-[*rel*-(2*S*;3*R*)-3-(2-chlorophenyl)-2-(2,4-difluorophenyl)-oxiranylmethyl]-2H-[1,2,4]triazole-3-thiol, 2-[2-chloro-4-(4-chlorophenoxy)phenyl]-1-(1,2,4-triazol-1-yl)pentan-2-ol, 1-[4-(4-chlorophenoxy)-2-(trifluoromethyl)phenyl]-1-cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol; imidazoles: imazalil, pefurazoate, prochloraz, triflumizol; pyrimidines, pyridines and piperazines: fenarimol, nuarimol, pyrifenox, triforine, [3-(4-chloro-2-fluoro-phenyl)-5-(2,4-difluorophenyl)isoxazol-4-yl]-(3-pyridyl)methanol;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methylpiperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)-ethanesulfonyl)-but-2-yl) carbamic acid-(4-fluorophenyl) ester;
   - compounds affecting cell membrane permeability and fatty acides: propamocarb
   - fatty acid amide hydrolase inhibitors: oxathiapiprolin;
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlorphenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1H,5H-[1,4]dithiino[2,3-c:5,6-c']dipyrrole-1,3,5,7(2H,6H)-tetraone;
I) Cell wall synthesis inhibitors
   - inhibitors of glucan synthesis: validamycin, polyoxin B; melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum,
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, tolprocarb, oxincopper, proquinazid, tebufloquin, tecloftalam, triazoxide, 2-butoxy-6-iodo-3-propylchromen-4-one, 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1H-pyrazol-1-yl]-1-[4-(4-{5-[2-fiuoro-6-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1H-pyrazol-1-yl]-1-[4-(4-{5-[2-chioro-6-(prop-2-yn-1-yloxy)phenyl]-4,5-dihydro-1,2-oxazol-3-yl}-1,3-thiazol-2-yl)piperidin-1-yl]ethanone, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxyacetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole), N-(6-methoxy-pyridin-3-yl) cyclopropanecarboxylic acid amide, 5-chloro-1-(4,6-dimethoxy-pyrimidin-2-yl)-2-methyl-1H-benzoimidazole, 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate, picarbutrazox , pentyl N-[6-[[(Z)-[(1-methyltetrazol-5-yl)-phenyl-methylene]amino]oxymethyl]-2-pyridyl]carbamate, 2-[2-[(7,8-difluoro-2-methyl-3-quinolyl)oxy]-6-fluoro-phenyl]propan-2-ol, 2-[2-fluoro-6-[(8-fluoro-2-methyl-3-quinolyl)oxy]phenyl]propan-2-ol, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline;
L) Biopesticides
L1) Microbial pesticides with fungicidal, bactericidal, viricidal and/or plant defense activator activity: *Ampelomyces quisqualis, Aspergillus flavus, Aureobasidium pullulans, Bacillus amyloliquefaciens, B. mojavensis, B. pumilus, B. simplex, B. solisalsi, B. subtilis, B. subtilis var. amyloliquefaciens, Candida oleophila*, *C. saitoana, Clavibacter michiganensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructicola, Microdochium dimerum, Microsphaeropsis ochracea*, *Muscodor albus*, *Paenibacillus polymyxa*, *Pantoea vagans*, *Phlebiopsis gigantea*, *Pseudomonas sp*., *Pseudomonas chloraphis*, *Pseudozyma flocculosa, Pichia anomala, Pythium oligandrum, Sphaerodes mycoparasitica, Streptomyces griseoviridis, S. lydicus*, *S. violaceusniger*, *Talaromyces flavus*, *Trichoderma asperellum, T. atroviride, T. fertile, T. gamsii, T. harmatum, T. harzianum; mixture of T. harzianum and T. viride; mixture of T. polysporum and T. harzianum; T. stromaticum, T. virens (also named Gliocladium virens), T. viride, Typhula phacorrhiza, Ulocladium oudemansii, Verticillium dahlia, zucchini yellow mosaic virus (avirulent strain);*
L2) Biochemical pesticides with fungicidal, bactericidal, viricidal and/or plant defense activator activity: *chitosan (hydrolysate), harpin protein, laminarin, Menhaden fish oil, natamycin, Plum pox virus coat protein, potassium or sodium bicarbonate, Reynoutria sachlinensis extract, salicylic acid, tea tree oil;*
L3) Microbial pesticides with insecticidal, acaricidal, molluscidal and/or nematicidal activity: *Agrobacterium radiobacter, Bacillus cereus, B. firmus, B. thuringiensis, B. thuringiensis ssp. aizawai, B. t. ssp. israelensis*, *B. t. ssp. galleriae, B. t. ssp. kurstaki, B. t. ssp. tenebrionis, Beauveria bassiana, B. brongniartii*, *Burkholderia sp., Chromobacterium subtsugae, Cydia pomonella granulosis virus, Cryptophlebia leucotreta granulovirus (CrleGV), Isaria fumosorosea, Heterorhabditis bacteriophora, Lecanicillium longisporum, L. muscarium (formerly Verticillium lecanii), Metarhizium anisopliae, M. anisopliae var. acridum, Nomuraea rileyi, Paecilomyces fumosoroseus, P. lilacinus*, *Paenibacillus popilliae, Pasteuria spp., P. nishizawae, P. penetrans, P. ramose, P. reneformis, P. thornea, P. usgae, Pseudomonas fluorescens, Steinernema carpocapsae, S. feltiae, S. kraussei;*
L4) Biochemical pesticides with insecticidal, acaricidal, molluscidal, pheromone and/or nematicidal activity: *L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, potassium silicate, sorbitol actanoate, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, Acacia negra extract, extract of grapefruit seeds and pulp, extract of Chenopodium ambrosiodae, Catnip oil, Neem oil, Quillay extract, Tagetes oil;*
L5) Microbial pesticides with plant stress reducing, plant growth regulator, plant growth promoting and/or yield enhancing activity: *Azospirillum amazonense A. brasilense, A.* *lipoferum, A. irakense, A. halopraeferens, Bradyrhizobium sp., B. elkanii, B. japonicum, B. liaoningense, B. lupini, Delftia acidovorans, Glomus intraradices, Mesorhizobium sp., Paenibacillus alvei, Penicillium bilaiae, Rhizobium leguminosarum bv. phaseolii, R. I. trifolii, R. I. bv. viciae, R. tropici, Sinorhizobium meliloti;*
L6) Biochemical pesticides with plant stress reducing, plant growth regulator and/or plant yield enhancing activity: *abscisic acid, aluminium silicate (kaolin), 3-decen-2-one, formononetin, genistein, hesperetin, homobrassinlide, humates, jasmonic acid or salts or derivatives thereof, lysophosphatidyl ethanolamine, naringenin, polymeric polyhydroxy acid, Ascophyllum nodosum (Norwegian kelp, Brown kelp) extract and Ecklonia maxima (kelp) extract;*
M) Growth regulators abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid, trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron, tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juve-noids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - ryanodine receptor inhibitors: chlorantraniliprole, cyantraniliprole, flubendiamide, N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-methylphenyl]-2-(3-chloro-2-pyridyl)-5-(trifluoromethyl)pyrazole-3-carboxamide; N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide; N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide; N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
   - others: benclothiaz, bifenazate, cartap, flonicamid, pyridalyl, pymetrozine, sulfur, thiocyclam, cyenopyrafen, flupyrazofos, cyflumetofen, amidoflumet, imicyafos, bistrifluron, pyrifluquinazon and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester.

The active, such as the pesticide has preferably a **melting point** of at least 30 °C, more preferably of at least 50 °C, and especially of at least 70 °C.

The active, such as the pesticide has preferably a **solubility in water** of less than 10 g/l at 20 °C, more preferably of less than 1 g/l, even more preferably of less than 0,5 g/l, and most preferably of less than 0,1 g/l.

The active, such as the pesticide may be present in any form, e.g. as liquid or solid. Preferably the pesticide is present in dispersed (e.g. suspended or emulsified) form, wherein the suspended form in more preferred.

Preferred pesticides are the fungicides of group B) Sterol biosynthesis inhibitors (SBI fungicides). More preferred pesticides are fungicides of the class of C14 demethylase inhibitors, such as the **triazoles**. In particular preferred are the fungicides selected from 2-[2-chloro-4-(4-chlorophenoxy)phenyl]-1-(1,2,4-triazol-1-yl)pentan-2-ol, 1-[4-(4-chlorophenoxy)-2-(trifluoromethyl)phenyl]-1-cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol.

The copolymers and the compositions according to the invention are particularly im-portant in various **cultivated plants**, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, rasp-berries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cu-cumber or melons; fiber plants, such as cotton, flax, hemp or jute; citrus fruit, such as oranges, lemons, grapefruits or mandarins; vegetables, such as spinach, lettuce, aspa-ragus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bana-nas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

The term **"plant propagation material"** is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil.

These young plants may also be protected before transplantation by a total or partial treatment by immersion or pouring.

The term "cultivated plants" is to be understood as including plants which have been modified by breeding, mutagenesis or genetic engineering including but not limiting to agricultural biotech products on the market or in development. Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

The present invention further relates to a method for **preparing the composition** accord-ing to the invention by mixing the active and the graft copolymer. The mixing of the components may be achieved by conventional equipment at any temperature, such as room temperature. Preferred mixing methods are those which are applied to prepare agrochemical compositions.

The present invention further relates to a **method for controlling** phytopathogenic fungi and/or undesired plant growth and/or undesired attack by insects or mites and/or for regulating the growth of plants, where the composition according to the invention is allowed to act on the particular pests, or the plants to be protected from the particular pest, the soil and/or on undesired plants and/or the useful plants. In general, the therapeutic treatment of humans and animals is excluded from the method for controlling phytopathogenic fungi and/or undesired vegetation and/or undesired attack by insects or mites and/or for regulating the growth of plants.

The graft copolymers and/or the composition according to the invention can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are **prepared** in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Examples for suitable **auxiliaries** are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable **solvents** and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, o-sec butyl phenol, cyclohexanol; glycols; DMSO; ketones, e.g. cyclohexanone; esters, e.g. lactates (such as 2-ethylhexyl lactate), carbonates, fatty acid esters, gamma-butyrolactone; fatty acids; phosphonates; amines; amides, e.g. N-methylpyrrolidone, fatty acid dimethylamides, N,N-dimethyl lactamide; and mixtures thereof.

Suitable **solid carriers** or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharide powders, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable **surfactants** are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable **anionic** surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable **nonionic** surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable **cationic** surfactants are quaternary surfactants, for example quaternary ammonium compounds with one or two hydrophobic groups, or salts of long-chain primary amines. Suitable **amphoteric** surfactants are alkylbetains and imidazolines. Suitable **block polymers** are block polymers of the A-B or A-B-A type comprising blocks of polyethylene oxide and polypropylene oxide, or of the A-B-C type comprising alkanol, polyethylene oxide and polypropylene oxide. Suitable **polyelectrolytes** are polyacids or polybases. Examples of polyacids are alkali salts of polyacrylic acid or polyacid comb polymers. Examples of polybases are polyvinylamines or polyethyleneamines.

Suitable **adjuvants** are compounds, which have a negligible or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5. Suitable **thickeners** are polysaccharides (e.g. xanthan gum, carboxymethylcellulose), anorganic clays (organically modified or unmodified), polycarboxylates, and silicates. Suitable **bactericides** are bronopol and isothiazolinone derivatives such as alkylisothiazolinones and benzisothiazolinones. Suitable **anti-freezing agents** are ethylene glycol, propylene glycol, urea and glycerin. Suitable **anti-foaming agents** are silicones, long chain alcohols, and salts of fatty acids. Suitable **colorants** (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants). Suitable **tackifiers or binders** are polyvinylpyrrolidons, polyvinylacetates, polyvinyl alcohols, polyacrylates, biological or synthetic waxes, and cellulose ethers.

Examples for composition types and their preparation are:
**i) Water-soluble concentrates (SL, LS)**
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
**ii) Dispersible concentrates (DC)**
   5-25 wt% of a compound I according to the invention and 1-10 wt% dispersant (e. g. polyvinylpyrrolidone) are dissolved in up to 100 wt% organic solvent (e.g. cyclohexanone). Dilution with water gives a dispersion.
**iii) Emulsifiable concentrates (EC)**
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
**iv) Emulsions (EW, EO, ES)**
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
**v) Suspensions (SC, OD, FS)**
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active substance. For FS type composition up to 40 wt% binder (e.g. polyvinylalcohol) is added.
**vi) Water-dispersible granules and water-soluble granules (WG, SG)**
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e. g. extrusion, spray tower, fluidized bed). Dilution with water gives a stable dispersion or solution of the active substance.
**vii) Water-dispersible powders and water-soluble powders (WP, SP, WS)**
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
**viii) Gel (GW, GF)**
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
**ix) Microemulsion (ME)**
   5-20 wt% of a compound **I** according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.
**x) Microcapsules (CS)**
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of a polyurea microcapsules. The monomers amount to 1-10 wt%. The wt% relate to the total CS composition.
**xi) Dustable powders (DP, DS)**
   1-10 wt% of a compound I according to the invention are ground finely and mixed intimately with up to 100 wt% solid carrier, e.g. finely divided kaolin.
**xii) Granules (GR, FG)**
   0.5-30 wt% of a compound I according to the invention is ground finely and associated with up to 100 wt% solid carrier (e.g. silicate). Granulation is achieved by extrusion, spray-drying or the fluidized bed.
**xiii) Ultra-low volume liquids (UL)**
   1-50 wt% of a compound I according to the invention are dissolved in up to 100 wt% organic solvent, e.g. aromatic hydrocarbon.

The compositions types i) to xiii) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical compositions generally comprise between 0.01 and 95%, preferably between 0.1 and 90%, and most preferably between 0.5 and 75%, by **weight of active substance**. The active substances are employed in a purity of from 90% to 100%, preferably from 95% to 100% (according to NMR spectrum).
Water-soluble concentrates (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of **treatment of plant propagation materials**, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying or treating compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the **amounts of active substances applied** are, depending on the kind of effect desired, from 0.001 to 2 kg per ha, preferably from 0.005 to 2 kg per ha, more preferably from 0.05 to 0.9 kg per ha, in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seed) are generally required.
When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (**tank mix**). These agents can be admixed with the compositions according to the invention in a weight ratio of 1:100 to 100:1, preferably 1:10 to 10:1.

The **user applies** the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a **kit or parts** of a binary or ternary mixture may be mixed by the user himself in a spray tank and further auxiliaries may be added, if appropriate.
In a further embodiment, either individual components of the composition according to the invention or partially **premixed** components, e. g. components comprising compounds I and/or active substances from the groups A) to O), may be mixed by the user in a spray tank and further auxiliaries and additives may be added, if appropriate.
In a further embodiment, either individual components of the composition according to the invention or partially premixed components, e. g. components comprising compounds I and/or active substances from the groups A) to O), can be **applied jointly** (e.g. after tank mix) or **consecutively.**

**Advantages** of the present invention are for example, that the composition according to the invention has an excellent storage stability (e.g. regarding particle size, viscosity). The graft copolymer increases the pesticidal activity of the pesticides. This adjuvant effect is achieved without decrease in the stability of the composition. The graft copolymer has a very low phytotoxicity. Such graft copolymers are easy to handle and have no adverse effects in contact to the skin, e.g. for the farmer handling the agrochemical formulations. The graft copolymer can easily produced in industrial scale for low costs. Further on, the graft polymer increases the retention of pesticides on the surface of plants. The graft polymer increases the uptake of pesticides into plants. The graft polymer combines various of the aforementioned advantages.

The invention is further illustrated but not limited by the following examples.

### Examples

- VP: N-vinyl pyrrolidone
- VAc: vinyl acetate
- EA: ethyl acetate solvent
- tBPPiv: tert. butyl perpivalate
- GPC: gel permeation chromatography
- Graft Polymer C-1:: Comparative graft polymer prepared from 13 wt% polyethylene glycol (average molecular weight 6000 g/mol), 57 wt% N-vinyl caprolactame, and 30 wt% vinyl acetate according to Example 1 of WO 2011/121477.
- Graft Polymer C-2:: Comparative graft polymer prepared according to Example 1 below from 60 wt% polyethylene glycol (average molecular weight 600 g/mol), 20 wt% vinyl acetate, and 20 wt% N-vinyl pyrrolidone.
- Graft Polymer C-3:: Comparative graft poylmer prepared according to Example 1 bewlo from 60 wt% APEG6 and 40 wt%vinylacetate (i.e. without N-vinyllactam). This polymer was not soluble in water. Thus, it was not possible to prepare suspensions concentrates or make the tests as decribed below.

### Alcohol alcoxylates:

- APEG 1:: ethoxylated saturated linear C16C18 fat alcohol, degree of ethoxylation about 80, melting point about 56 °C, HLB value about 18,5.
- APEG 2:: ethoxylated saturated linear C16C18 fat alcohol, degree of ethoxylation about 25, melting point about 46 °C, HLB value about 16.
- APEG 4:: ethoxylated linear C12C14 fat alcohol, degree of ethoxylation about 7, HLB value about 12.
- APEG 5:: ethoxylated saturated iso-C13 alcohol, degree of ethoxylation about 20, melting point about 36 °C, HLB about 16,5.
- APEG 6:: ethoxylated saturated linear C13C15 alcohol, degree of ethoxylation about 11, HLB about 14.

### Example 1 - Graft copolymer preparation

The reaction vessel containing 100 g APEG 1 and 25 g EA was gassed with nitrogen and heated to 77 °C. Then one part of feed 2 (12,2 g tBPPiv, 50 g EA) was added and the mixture stirred for 15 min. Then feed 1 (160g VAc, 160 g VP, 120 g EA, 2,2 g 2-mercapto ethanol) and the rest of feed 2 were introduced in the reaction mixture. Feed 1 was introduced in 5 h, feed 2 was introduced in the course of 5,5 h. The reaction mixture was then kept at 77 °C for additional 3 h. Then feed 3 (200 g EA) was introduced and the reacture mixture was cooled down. Thereafter EA was distilled of and the reaction mixture was subject to steam distillation. Graft polymer A was obtained. The resulting polymer solution of Graft Polymer A in water was a clear, yellow solution. GPC measurements showed Mₙ = 2.894 g/mol and M_{w} = 6.514 g/mol.

The preparation of Graft Polymers B-F was carried out analogously.

**Table 1:**

| | |
|---|---|
| Polymer B | 200 g APEG 2 |
| | 100g Vac |
| | 100 g VP |
| | 8 g tBPPiv |
| | 2 g mercapto ethanol |
| | 395 g EA |
| Polymer C | 200 g APEG 4 |
| | 100g Vac |
| | 100 g VP |
| | 8 g tBPPiv |
| | 2 g mercaptoethanol |
| | 395 g EA |
| Polymer D | 240 g APEG 5 |
| | 80g Vac |
| | 80 g VP |
| | 8,5 g tBPPiv |
| | 1,6 g mercapto ethanol |
| | 395 g EA |
| Polymer E | 240 g APEG 6 |
| | 80 g Vac |
| | 80 g VP |
| | 8,5 tBPPiv |
| | 1,6 g mercapto ethanol |
| | 395 g EA |
| Polymer F | 240 g APEG 4 |
| | 80 g Vac |
| | 80 g VP |
| | 8,5 tBPPiv |
| | 1,6 g mercapto ethanol |
| | 395 g EA |
| Polymer G | 200 g APEG 6 |
| | 100 g Vac |
| | 100 g VP |
| | 10,5 g tBPPiv |
| | 2 g 2-mercapto ethanol |
| | 415 g AE |
| Polymer H | 240 g APEG 6 |
| | 100 g Vac |
| | 60 g VP |
| | 4,6 g tBPPiv |
| | 0,8 g 2-mercapto ethanol |
| | 395 g EA |
| Polymer I | 280 g APEG 6 |
| | 60 g Vac |
| | 60 g VP |
| | 6,4 g tBPPiv |
| | 1,2 g 2-mercapto ethanol |
| | 480 g EA |
| Polymer K | 240 g APEG 2 |
| | 80 g Vac |
| | 80 g VP |
| | 6,1 g tBPPiv |
| | 1,1 g 2-mercapto ethanol |
| | 570 g EA |
| Polymer M | 240 g APEG 6 |
| | 80 g Vac |
| | 80 g VP |
| | 4,6 g tBPPiv |
| | 0,8 g 2-mercapto ethanol |
| | 320 g EA |

### Example 2 - Increased efficacy - Comparative data

An aqueous suspension concentrate ("SC1") was prepared containing 300 g/l fluxapyroxad (a fungicide, water solubility 4 mg/L at 20 °C) and common auxiliaries (such as 1,2-propylene glycol, anionic phenolsulfonic acid-urea-formaldehyde condensate surfactant, antibacterial agent, antifoaming agent, thickener).
The pesticidal activity was tested in greenhouse tests on wheat variety Monopol, which were infected with the fungi *Puccinia Recondata*/*Tritici.* The plants were treated with SC1 three days after the inoculation at a use rate of 25, 8.25 or 2.75 g pesticide per ha (200 l water/ha). The use rate of the adjuvants was kept constant at 250 g per ha. The percentage of the infected leaf surface ares (7 days after inoculation) was summarized in Table 2A.

The data showed that the composition with the graft polymer according to the invention has a higher pesticidal activity compared to the control without graft polymer or with the comparative graft polymer C-1 (based on a grafted polyethene glycol instead of a grafted alcohol ethoxylate).

**Table 2A:**

| Pesticide rate | 25 g/ha | 8.25 g/ha | 2.75 g/ha |
|---|---|---|---|
| | % Infection | % Infection | % Infection |
| Untreated control ^{a)} | 90 | 90 | 90 |
| SC1 ^{a)} | 90 | 89 | 90 |
| SC1 with Graft Polymer C-1 ^{a)} | 88 | 89 | 90 |
| SC1 with Graft Polymer E | 1 | 8 | 72 |

| | | | |
|---|---|---|---|
| a)Comparative, not according to the invention. | | | |

The above greenhouse tests were also made with a suspension concenctrate "SC2" of the triazole fungicide 2-[4-(4-chlorophenoxy)-2-(trifluoromethyl)phenyl]-1-(1H-1,2,4-triazol-1-yl)propan-2-ol (100 g/ha) instead of fluxapyroxad. The results are summarized in Table 2B:

**Table 2B:**

| Pesticide rate | 25 g/ha | 8.25 g/ha | 2.75 g/ha |
|---|---|---|---|
| | % Infection | % Infection | % Infection |
| Untreated control ^{a)} | 90 | 90 | 90 |
| SC2 ^{a)} | 90 | 90 | 89 |
| SC2 with Graft Polymer C-1 ^{a)} | 86 | 89 | 90 |
| SC2 with Graft Polymer E | 2 | 4 | 10 |

| | | | |
|---|---|---|---|
| a)Comparative, not according to the invention. | | | |

### Example 3 - Increased efficacy of various graft polymers

The aqueous suspensions concentracte SC1 containing 300 g/l fluxapyroxad was tested as described in Example 2 with various graft polymer from Example 1. The results are summarized in Table 3:

**Table 3:**

| Pesticide rate | 25 g/ha | 8.25 g/ha |
|---|---|---|
| | % Infection | % Infection |
| Untreated control ^{a)} | 89 | 89 |
| SC1 ^{a)} | 83 | 81 |
| SC1 with Graft Polymer E | 0 | 6 |
| SC1 with Graft Polymer G | 2 | 11 |
| SC1 with Graft Polymer H | 3 | 6 |
| SC1 with Graft Polymer N | 1 | 4 |
| SC1 with Graft Polymer I | 1 | 7 |

| | | |
|---|---|---|
| a)Comparative, not according to the invention. | | |

### Example 4 - Increased efficacy of various graft polymers

The aqueous suspensions concentracte SC1 containing 300 g/l fluxapyroxad was tested as described in Example 2 with various graft polymer from Example 1. The results are summarized in Table 4:

**Table 4:**

| Pesticide rate | 25 g/ha | 8.25 g/ha |
|---|---|---|
| | % Infection | % Infection |
| Untreated control ^{a)} | 80 | 80 |
| SC1 ^{a)} | 83 | 80 |
| SC1 with Graft Polymer K | 10 | 26 |
| SC1 with Graft Polymer F | 6 | 15 |

| | | |
|---|---|---|
| a)Comparative, not according to the invention. | | |

### Example 5 - Increased uptake and retention of pesticide in leaves

An aqueous suspension concentrate ("SC3") was prepared containing 300 g/l fluxapyroxad, 1,2-propylene glycol, anionic phenolsulfonic acid-urea-formaldehyde condensate surfactant, sodium salt of naphthalene sulfonate condensate, antibacterial agent, antifoaming agent, thickener. The spray mixture was applied at a rate of 200 l/ha, 12,5 g/ha pesticide and 250 g/ha graft polymer.

The uptake of the pesticide in the leave was determined as decribed by Berghaus R, Nolte M, Reinold A 2010. "Optimization of agrochemical formulations by adjuvants using lab track sprayer and HPLC-MS-MS analysis". In: Baur P and Bonnet M ed. Proc. 9th Intern. Symp. on Adjuvants for Agrochemicals. ISAA 2010 Freising, Germany. Pp. 239-244: Wheat plants (Triticum aestivum variety Mel*on*) were used. Subsequently to spraying, the plants were cultivated again in the greenhouse under ambient conditions. After 8 days samples of 10 - 15 treated leaves were cut off and weighed. Leaves were cut into small pieces, and washed with 50 % methanol in demineralized water as washing medium for 5 min. Then, the washing medium was separated from the leaves. The leaves were washed again with washing medium for 5 min. Both washing media were combined and diluted for analysis.

Finally, the leaves were transferred to a vial containing the extraction medium (75 % methanol, 20 % water and 5 % HCI) and homogenized using a Polytron PT 6100 dispersing unit (Kinematica, CH) for 2 min. 10 ml of the extract were centrifuged with 4000 rpm for 5 min. 2 ml of the supernatant were treated with 2 ml NaOH (0.2 mol/L) and 5 ml cyclohexane, and stirred for 30 min and centrifuged subsequently. 1 ml of the cyclohexane phase was transferred to a glass vial and dried (Liebisch N₂ Evaporator, Germany). The residue was solubilized in methanol/water 50:50 and analyzed by HPLC-MS/MS. In addition, unsprayed plants were treated in the same way to see whether they are contaminated. Unsprayed leaves were spiked with standard active ingredient to determine the recovery of active ingredient during washing and extracting steps. According to the recovery rate the measured sample values were corrected. Retention (total amount of active found in and on the plant) is equal to the sum of active concentrations found during washing and extracting steps.

The results that are given in Table 5 showed that the usage of invented adjuvants drastically increases the retention and the uptake of pesticide.

The comparative Graft Polymer C-1 and C2 are based on an hydrogen-terminated alkoxylate instead of an alcohol alkoxylate. The data showed that this modification resulted in an increased uptake and retention of the active.

**Table 5**

| | Uptake (mg/kg leave) | Retention (mg/kg leave) |
|---|---|---|
| SC1 ^{a)} | 0.25 | 2.61 |
| SC1 with Graft Polymer E | 2.38 | 8.62 |
| SC1 with Graft Polymer G | 1.88 | 9.19 |
| SC1 with Graft Polymer M | 1.44 | 6.95 |
| SC1 with Graft Polymer H | 1.74 | 10.03 |
| SC1 with Graft Polymer N | 1.63 | 7.58 |
| SC1 with Graft Polymer I | 2.49 | 9.86 |
| SC1 with Graft Polymer C-1 ^{a)} | 0.6 | 4.5 |
| SC1 with Graft Polymer C-2 ^{a)} | 0.5 | 3.9 |

| | | |
|---|---|---|
| a)Comparative, not according to the invention. | | |

### Example 6 - Increased storage stability of suspension concentrate

An aqueous suspension concentrate "SC4" was prepared comprising 80 g/l fluxapyroxad, 80 g/l graft polymer (see Table 6), 25 g/l 1,2-propylene glycol, 13 g/L sodium salt of naphthalene sulfonate condensate, 1,5 g/l xanthan gum, 5 g/l anionic phenolsulfonic acid-urea-formaldehyde condensate, silicon defoamer, and antibacterials.

For comparison, the aqueous suspension concentrate "SC4" was prepared without the addition of any graft polymer ("SC4 without graft polymer")
The suspension concentrates were stored for 14 days at 20 °C or at 40 °C. The stability of the formulation was determined by using instrument Malvern. D₉₀ is the value in µm that 90 % (volume /volume) of the particles existing in the formulation have a size smaller this value. An increase in D 90 is an indication for the destabilization of SC formulations.

**Table 6: Particle size D₉₀ during storage of suspension concentrate "SC4"**

| Polymer added | 0 d | 14 d at 20 °C | 14 d at 40 °C |
|---|---|---|---|
| Polymer E | 2.0 µm | 2.0 µm | 1.9 µm |
| Polymer K | 2.0 µm | 2.0 µm | 1.9 µm |
| Polymer F | 2.0 µm | 2.0 µm | 1.9 µm |
| Polymer L | 2.0 µm | 1.9 µm | 2.0 µm |
| Polymer G | 2.0 µm | 2.0 µm | 2.0 µm |
| Polymer M | 2.0 µm | 2.0 µm | 2.0 µm |
| Alcohol Alkoxylate A ^{a)} | 2.0 µm | 6.5 µm | 8.2 µm |
| Alcohol Alkoxylate B ^{a)} | 2.0 µm | 7.9 µm | 7.8µm |

| | | | |
|---|---|---|---|
| a)Comparative data | | | |

For comparison, the Alcohol Alkoxylate A (a C₁₂₋₁₈ fatty alcohol, ethoxylated and propoxylated, 1-2.5 EO and 1-2.5 PO) and Alcohol Alkoxylate B (a fatty alcohol alkoxylate, surface tension about 30 mN/m (1g/l at 23 °C), viscosity about 75 mPas (Brookfield, at 23 °C)) were used. These comparative polymers resulted in an clear increase of particle size during storage.

### Example 7 - Solubilization of pesticide

Solubilization measurements were carried out with a highthroughput screening robot. This setup dosed 10 mg solid active and 500 µm of the respective 3wt% liquid polymer solution (in Cl-PAC water D) into wells on a micro titer plate. After the addition of stirring bars and 24 hours incubation time the samples were filtrated via polypropylene filters in order to separaate dissolved active and its solid form ated. The amount of solubilized active was determined via UV/VIS spectroscopy. The solubilities of various actives were summarized in Tables 7-11.

**Table 7: Increased solubility of Xemium® (fluxapyroxad)**

| | Solubility in ppm |
|---|---|
| Active without polymer | 10 |
| Polymer E | 1253 |
| Polymer K | 910 |
| Polymer F | 1414 |
| Polymer G | 535 |
| Polymer M | 551 |

**Table 8: Increased solubility of the triazole fungicide 2-[4-(4-chlorophenoxy)-2-(trifluoromethyl)phenyl]-1-(1H-1,2,4-triazol-1-yl)propan-2-ol**

| | Solubility in ppm |
|---|---|
| Active without polymer | 15 |
| Polymer E | 1653 |
| Polymer F | 2167 |
| Polymer I | 1538 |

**Table 9: Increased solubility of fipronil**

| | Solubility in ppm |
|---|---|
| Active without polymer | 12 |
| Polymer E | 701 |
| Polymer F | 821 |
| Polymer I | 785 |

**Table 10: Increased solubility of fenofibrate**

| | Solubility in ppm |
|---|---|
| Active without polymer | 0 |
| Polymer E | 377 |
| Polymer F | 467 |
| Polymer I | 296 |

**Table 11: Increased solubility of cabamazepine**

| | Solubility in ppm |
|---|---|
| Active without polymer | 181 |
| Polymer E | 1648 |
| Polymer F | 1668 |
| Polymer I | 1312 |

## Claims

1. A composition comprising an active and a graft copolymer obtainable by free-radical polymerization of a monomer mixture comprising N-vinyllactam, vinyl ester, and an alcohol alkoxylate, wherein the N-vinyl lactam is N-vinyl pyrrolidon.

2. The composition according to claim 1, where the graft copolymer is obtainable from a monomer mixture comprising
i) 5 to 60% by weight of N-vinyllactam,
ii) 5 to 60% by weight of vinyl ester,
iii)10 to 80% by weight of alcohol alkoxylate, and
iv)up to 20% by weight of additional comonomers,
wherein the sum of component i) to iv) adds up to 100 %.

3. The composition according to any of claims 1 to 2, where the vinyl ester is vinyl acetate, vinyl propionate, or mixtures thereof.

4. The composition according to any of claims 1 to 3, where the alcohol alkoxylate comprises 3 to 100 units of alkoxylate.

5. The composition according to any of claims 1 to 4, where the alcohol alkoxylate is an alcohol ethoxylate.

6. The composition according to any of claims 1 to 5, where the alcohol alkoxylate is based on a linear or branched, saturated or unsaturated C₈-C₃₄ alcohol.

7. The composition according to any of claims 1 to 6, where the amount of the graft polymer is in the range of from 10 to 500 wt%, based on the weight of the active.

8. The composition according to any of claims 1 to 7, where the active has a solubility in water of less than 10 g/l at 20 °C.

9. The composition according to any of claims 1 to 8, where the active is a pesticide or a pharmaceutical active.

10. The composition according to any of claims 1 to 9, where the weight ratio of the N-vinyllactam to the vinyl ester is from 3 : 1 to 1 : 3.

11. The composition according to any of claims 1 to 10, where the graft copolymer is obtainable from a monomer mixture comprising
i) 10 to 45% by weight of N-vinyllactam,
ii) 10 to 45% by weight of vinyl ester,
iii)15 to 80% by weight of a alcohol alkoxylate, and
iv)up to 10% by weight of additional comonomers,
wherein the sum of component i) to iv) adds up to 100 %.

12. A graft copolymer as defined in any of claims 1 to 11.

13. A method for preparing the composition as defined in any of claims 1 to 11 by mixing the active and the graft copolymer.

14. A method for controlling phytopathogenic fungi and/or undesired plant growth and/or undesired attack by insects or mites and/or for regulating the growth of plants, where the composition as defined in any of claims 1 to 11 is allowed to act on the particular pests, or the plants to be protected from the particular pest, the soil and/or on undesired plants and/or the useful plants, wherein the therapeutic treatment of humans and animals is excluded.

## Patentansprüche

1. Zusammensetzung, umfassend einen Wirkstoff und ein durch radikalische Polymerisation einer N-Vinyllactam, Vinylester und ein Alkoholalkoxylat umfassenden Monomermischung erhältliches Pfropfcopolymer, wobei es sich bei dem N-Vinyllactam um N-Vinylpyrrolidon handelt.

2. Zusammensetzung nach Anspruch 1, wobei das Pfropfcopolymer aus einer
i) 5 bis 60 Gew.-% N-Vinyllactam,
ii) 5 bis 60 Gew.-% Vinylester,
iii) 10 bis 80 Gew.-% Alkoholalkoxylat und
iv) bis zu 20 Gew.-% zusätzliche Comonomere umfassenden Monomermischung erhältlich ist,
wobei die Summe der Komponenten i) bis iv) 100% beträgt.

3. Zusammensetzung nach einem der Ansprüche 1 bis 2, wobei es sich bei dem Vinylester um Vinylacetat, Vinylpropionat oder Mischungen davon handelt.

4. Zusammensetzung nach einem der Ansprüche 1 bis 3, wobei das Alkoholalkoxylat 3 bis 100 Alkoxylateinheiten umfasst.

5. Zusammensetzung nach einem der Ansprüche 1 bis 4, wobei es sich bei dem Alkoholalkoxylat um ein Alkoholethoxylat handelt.

6. Zusammensetzung nach einem der Ansprüche 1 bis 5, wobei das Alkoholalkoxylat auf einem geradkettigen oder verzweigten, gesättigen oder ungesättigten C₈-C₃₄-Alkohol basiert.

7. Zusammensetzung nach einem der Ansprüche 1 bis 6, wobei die Menge an Pfropfpolymer im Bereich von 10 bis 500 Gew.-% liegt, bezogen auf das Gewicht des Wirkstoffs.

8. Zusammensetzung nach einem der Ansprüche 1 bis 7, wobei der Wirkstoff bei 20°C eine Löslichkeit im Wasser von weniger als 10 g/l aufweist.

9. Zusammensetzung nach einem der Ansprüche 1 bis 8, wobei es sich bei dem Wirkstoff um ein Pestizid oder einen pharmazeutischen Wirkstoff handelt.

10. Zusammensetzung nach einem der Ansprüche 1 bis 9, wobei das Gewichtsverhältnis von N-Vinyllactam zum Vinylester 3:1 bis 1:3 beträgt.

11. Zusammensetzung nach einem der Ansprüche 1 bis 10, wobei das Pfropfcopolymer aus einer
i) 10 bis 45 Gew.-% N-Vinyllactam,
ii) 10 bis 45 Gew.-% Vinylester,
iii) 15 bis 80 Gew.-% eines Alkoholalkoxylats und
iv) bis zu 10 Gew.-% zusätzlicher Comonomere umfassenden Monomermischung erhältlich ist,
wobei die Summe der Komponenten i) bis iv) 100% beträgt.

12. Pfropfcopolymer, definiert wie in einem der Ansprüche 1 bis 11.

13. Verfahren zur Herstellung der wie in einem der Ansprüche 1 bis 11 definierten Zusammensetzung durch Mischen des Wirkstoffs mit dem Pfropfcopolymer.

14. Verfahren zur Bekämpfung phytopathogener Pilze und/oder unerwünschten Pflanzenwuchses und/oder eines unerwünschten Befalls durch Insekten oder Milben und/oder zum Regulieren des Wachstums von Pflanzen, wobei die wie in einem der Ansprüche 1 bis 11 definierte Zusammensetzung auf die jeweiligen Schädlinge oder die vor dem jeweiligen Schädling zu schützenden Pflanzen, den Boden und/oder auf unerwünschte Pflanzen und/oder die Nutzpflanzen einwirken gelassen wird, wobei die therapeutische Behandlung von Menschen und Tieren ausgeschlossen ist.

## Revendications

1. Composition comprenant un principe actif et un copolymère greffé pouvant être obtenu par polymérisation radicalaire d'un mélange de monomères comprenant du N-vinyllactame, un ester vinylique, et un alcoxylate d'alcool, où le N-vinyllactame est la N-vinylpyrrolidone.

2. Composition selon la revendication 1, dans laquelle le copolymère greffé peut être obtenu à partir d'un mélange de monomères comprenant
i) de 5 à 60% en poids de N-vinyllactame,
ii) de 5 à 60% en poids d'un ester vinylique,
iii) de 10 à 80% en poids d'un alcoxylate d'alcool, et
iv) jusqu'à 20% en poids de co-monomères supplémentaires,
où la somme des composants i) à iv) totalise 100%.

3. Composition selon l'une quelconque des revendications 1 à 2, dans laquelle l'ester vinylique est l'acétate de vinyle, le propionate de vinyle, ou des mélanges de ceux-ci.

4. Composition selon l'une quelconque des revendications 1 à 3, dans laquelle l'alcoxylate d'alcool comprend de 3 à 100 motifs d'alcoxylate.

5. Composition selon l'une quelconque des revendications 1 à 4, dans laquelle l'alcoxylate d'alcool est un éthoxylate d'alcool.

6. Composition selon l'une quelconque des revendications 1 à 5, dans laquelle l'alcoxylate d'alcool est à base d'un C₈-C₃₄-alcool linéaire ou ramifié, saturé ou insaturé.

7. Composition selon l'une quelconque des revendications 1 à 6, dans laquelle la quantité de polymère greffé se trouve dans la plage allant de 10 à 500% en poids, sur la base du poids du principe actif.

8. Composition selon l'une quelconque des revendications 1 à 7, dans laquelle le principe actif possède une solubilité dans l'eau inférieure à 10 g/l à 20°C.

9. Composition selon l'une quelconque des revendications 1 à 8, dans laquelle le principe actif est un pesticide ou un principe actif pharmaceutique.

10. Composition selon l'une quelconque des revendications 1 à 9, dans laquelle le rapport pondéral du N-vinyllactame à l'ester vinylique va de 3:1 à 1:3.

11. Composition selon l'une quelconque des revendications 1 à 10, dans laquelle le copolymère greffé peut être obtenu à partir d'un mélange de monomères comprenant
i) de 10 à 45% en poids de N-vinyllactame,
ii) de 10 à 45% en poids d'un ester vinylique,
iii) de 15 à 80% en poids d'un alcoxylate d'alcool, et
iv) jusqu'à 10% en poids de co-monomères supplémentaires,
où la somme des composants i) à iv) totalise 100%.

12. Copolymère greffé tel que défini selon l'une quelconque des revendications 1 à 11.

13. Méthode de préparation de la composition telle que définie selon l'une quelconque des revendications 1 à 11, par le mélange du principe actif et du copolymère greffé.

14. Méthode de contrôle de champignons phytopathogènes et/ou d'une croissance de plantes non désirée et/ou d'une attaque non désirée par des insectes ou des acariens et/ou de régulation de la croissance de plantes, dans laquelle on permet à la composition telle que définie selon l'une quelconque des revendications 1 à 11 d'agir sur les nuisibles particuliers, ou les plantes devant être protégées contre le nuisible particulier, le sol et/ou sur les plantes non désirées et/ou les plantes utiles, où le traitement thérapeutique d'êtres humains et d'animaux est exclu.
